# EUROPEAN PATENT APPLICATION

(11) **EP 1 693 470 A1**
(43) Date of publication of application: **23.08.2006**
(21) Application number: 06250812.2
(22) Date of filing: 15.02.2006
(51) Int. Cl.: C12Q 1/68

(54) **Methods and compositions for determining cytotoxicity of transfection agents**

(30) Priority: 15.02.2005 US 59209; 10.06.2005 US 150409
(71) Applicant: Agilent Technologies, Inc., Palo Alto, CA 94306 (US)
(72) Inventor: Ilsley, Diane D., Loveland, CO 80537-0599 (US); Bhattacharjee, Arindam, Loveland, CO 8053-0599 (US); Anderson, Emily M., Lafayette, CO 80026 (US); Khvovova, Anastasia, Boulder, CO 80305 (US)
(74) Representative: Ellis, Katherine Elizabeth Sleigh

(57) **Abstract**

Methods and compositions for determining cytotoxicity of a transfection agent are provided. In one aspect, a cell is contacted with a transfection agent. Following contact, a cytotoxic marker profile of the cell is evaluated to determine whether the transfection agent has mediated a cytotoxic response in the cell. Also provided are compositions and reagents, and kits for practicing the subject methods. The subject methods and compositions find use in a variety of different applications.

## Description

This invention relates to methods and compositions for determining cytotoxicity, and in particular to determining non-specific cytotoxicity of a transfection agent.

The introduction of exogenous nucleic acids into cells has proven to be a powerful tool in biological research and is used for many purposes, including expressing a specific gene product in a cell, measuring the activity of a specific cellular process, and activating or inhibiting the expression of a specific cellular gene. In some applications, expression of a specific gene product (e.g., an RNA transcript, a protein, etc.) in cells is done to gain an understanding of its biological, or functional, activity. The protein expressed from a nucleic acid vector introduced into a target cell may also be purified from the target cell and used in other applications (e.g., for therapeutics). Alternatively, the nucleic acid introduced can function to inhibit the translation of a specific gene to elucidate and/or inhibit its function (e.g., RNAi or antisense oligonucleotides). In still other applications, the nucleic acid may be designed to function as an indicator for a specific activity, as in reporter gene vectors that measure specific transcriptional responses. In gene therapy applications, the introduction of nucleic acids into cells is requisite, as the goal of this therapeutic approach is to replace an absent or defective gene of a cell (e.g., replacing the common gamma chain gene in X-linked SCID patients) or imparting a functionality to the cell that it is unable to provide for itself (e.g., enhanced resistance to chemotherapeutic agents).

The introduction of nucleic acids into cells has been accomplished using a variety of agents and methods. Viruses that have been engineered to encode the desired nucleic acid have proven to be very efficient at nucleic acid delivery. However, these systems are somewhat complex given that the expression of viral genes still present in the vector may have unintended consequences. Alternatively, "naked" nucleic acids can be introduced into cells in a process called transfection, which can be accomplished a number of ways. Methods include precipitating nucleic acids onto cells with calcium phosphate or DEAE dextran, introducing nucleic acid-permeable pores in cells using electric pulses (electroporation), or complexing the nucleic acids with chemical compounds that facilitate cellular uptake.

Lipid-based transfection agents have proven useful for nucleic acid transfection into cells. These lipid-based transfection agents form complexes with nucleic acids and mediate cellular entry when placed in contact with target cells. Lipid-based transfection agents are simple to use, efficient, and allow one to introduce virtually any nucleic acid into cells, thereby eliminating the need to include "extra" domains or sequences as is required in viral vectors.

As with viral vectors, however, lipid-based transfection agents can induce unintended effects in target cells. These cytotoxic effects can interfere with the accurate interpretation of experimental results and/or lead to cell death.
Therefore, it is desirable to detect cytotoxic effects that may be induced by the lipid-based transfection agent itself and not the exogenous nucleic acid, so that the experimental results can be interpreted more accurately.

Previous methods to assay for transfection agent induced cytotoxicity have focused on testing cell viability and include measuring the release of an internalized radioactive element (e.g., ⁵¹Cr release assay), observing the ability of target cells to actively exclude dyes (e.g., Trypan Blue), and measuring the activity of cellular enzymes released in the media upon cell death (e.g., LDH activity). However, these assays do not distinguish between cell death mediated by a specific nucleic acid sequence being introduced (e.g., because of a transcript and/or protein product it encodes or because the nucleic acid molecule itself binds to a cellular molecule, interfering with that molecule's function) and non-specific cytotoxic effects (e.g., due to experimental conditions, the transfection agent itself, or non-specific effects of the nucleic acid not associated with its sequence) that can adversely impact the outcome of a nucleic acid transfection.

As such, there is a continued need for the development of new methods for determining non-specific transfection agent-mediated cytotoxicity other than assaying for cell death.

According to a first aspect of the present invention, there is provided a method of determining whether a transfection agent-mediated cytotoxic response has occurred in a cell as specified in claim 1.

According to a second aspect of the present invention, there is provided an array of probes immobilised on a solid support as specified in claim 8.

According to a third aspect of the present invention, there is provided a kit as specified in claim 9.

According to a fourth aspect of the present invention, there is provided a collection of gene-specific primers as specified in claim 10.

Methods and compositions for determining cytotoxicity of a transfection agent are provided. In one embodiment, a target cell is contacted with a transfection agent. Following contact, a cytotoxic marker profile, e.g., a cytotoxic marker gene expression profile, is evaluated to determine whether the transfection agent has mediated a cytotoxic response in the cell. Also provided are compositions and reagents, kits and systems that may be used to practice the subject methods. The subject methods and compositions find use in a variety of different applications.

As such, in representative embodiments the invention provides a method of determining whether a transfection agent-mediated non-specific cytotoxic response has occurred in a cell, where the method includes: (a) contacting the cell with a transfection agent; and (b) evaluating a cytotoxic marker profile of at least one cytotoxic marker expressed by the cell to determine whether the transfection agent-mediated cytotoxic response has occurred in the cell. In certain embodiments, the cytotoxic marker profile is a profile of at least two different cytotoxic markers. In certain embodiments, at least one of the cytotoxic markers is an expression product of a gene listed in Table 1, e.g., where the cytotoxic marker profile is a cytoxic marker expression profile.

In certain embodiments, the method includes obtaining an expression profile, where the expression profile may be a nucleic acid expression profile or a polypeptide expression profile, or a combination thereof. In certain embodiments, the transfection agent is complexed with a nucleic acid, where the nucleic acid may be a deoxyribonucleic acid or a ribonucleic acid, analog (e.g., PNA or LNA molecule) or derivative thereof, or combinations thereof, and in certain embodiments is a RNAi agent, which may be a deoxyribonucleic acid or a ribonucleic acid. In other embodiments, the method further includes determining whether a RNAi-mediated interferon response has occurred in the cell. In certain embodiments, the method is employed to evaluate data obtained from a gene-silencing assay, e.g., a RNAi gene-silencing assay. In certain embodiments, the evaluating step occurs at least about 24 hours following the contacting step.

Also provided is a set of of probes (e.g., an array of probes). In one aspect, the probes are immobilized on a solid support in the form of an array, where the array includes at least one transfection agent-mediated non-specific cytotoxicity probe ("cytotoxicity probe") (e.g., a binding partner that specifically binds under the assay conditions used to detect the presence of a cytotoxic marker). The cytoxicity probe may be a nucleic acid, a polypeptide (e.g., antibody, a ligand) etc. In one aspect, the cytoxicity probe binds to or otherwise enables detection of a gene product expressed by a gene listed in Table 1, and in certain embodiments includes at least two different cytotoxicity probes, each corresponding to a different gene listed in Table 1. In certain embodiments, the probe is a polypeptide, while in other embodiments the probe is a nucleic acid.

Also provided are kits that include at least one cytotoxicity probe that specific binds to a cytotoxic marker. In one aspect, the kits include instructions for using this element in a method of determining whether a transfection agent mediated cytotoxic response has occurred in a cell. In certain embodiments, the genomic domain expression evaluation element is an array or is part of an array as described above. In certain embodiments, the kit also includes gene-specific primers specific for at least two of the genes of Table 1. In certain embodiments, the kits further include a transfection agent. In certain embodiments, the kits further include a cell.

Also provided is a collection of gene-specific primers that includes gene-specific primers for at least two genes listed on Table 1.

For convenience, certain terms employed in the specification, examples, and appended claims are collected here.

As used herein, the term "vector" refers to a nucleic acid molecule capable of transporting another nucleic acid to which it has been linked from a first location to a second location, e.g., from an extracellular to an intracellular location. One type of vector is a genomic integrated vector, or "integrated vector", which can become integrated into the chromosomal DNA of the host cell. Another type of vector is an episomal vector, e.g., a nucleic acid capable of extra-chromosomal replication in an appropriate host, e.g., a eukaryotic or prokaryotic host cell. Vectors capable of directing the expression of genes to which they are operatively linked are referred to herein as "expression vectors". In the present specification, "plasmid" and "vector" are used interchangeably unless otherwise clear from the context. Methods which are well known to those skilled in the art can be used to construct various plasmids and vectors; see, for example, the techniques described in Sambrook et al., Molecular Cloning A Laboratory Manual, Cold Spring Harbor Laboratory (2001) N.Y.

As used herein, the term "gene" or "recombinant gene" refers to a nucleic acid comprising an open reading frame encoding a polypeptide, including exon and (optionally) intron sequences. The term "intron" refers to a DNA sequence present in a given gene that is not translated into protein and is generally found between exons in a DNA molecule. In addition, a gene may optionally include its natural promoter (, i.e., the promoter with which the exons and introns of the gene are operably linked in a non-recombinant cell, i.e., a naturally occurring cell), and associated regulatory sequences, and may or may not have sequences upstream of the AUG start site, and may or may not include untranslated leader sequences, signal sequences, downstream untranslated sequences, transcriptional start and stop sequences, polyadenylation signals, translational start and stop sequences, ribosome binding sites, and the like.

A "protein coding sequence" or a sequence that "encodes" a particular polypeptide or peptide, is a nucleic acid sequence that is transcribed (in the case of DNA) and is translated (in the case of mRNA) into a polypeptide in vitro or in vivo when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from viral, procaryotic or eukaryotic mRNA, genomic DNA sequences from viral, procaryotic or eukaryotic DNA, and even synthetic DNA sequences. A transcription termination sequence may be located 3' to the coding sequence.

As used herein, the term "transfection" means the introduction of cargo agent, e.g., a nucleic acid, such as an expression vector, antisense agent, and RNAi agent, etc., into a recipient cell. In one aspect, "transfection" includes methods that employ transfection agents that enhance efficiency of delivery of the cargo agent into the target cell.

A "transfection agent" is any of a class of chemical compounds that find use in increasing the efficiency of cargo, e.g., nucleic acid, transfer into a target cell. An agent is considered to increase efficiency of cargo transfer into a target cell, and therefore considered to be a transfection agent, when the amount of cargo, e.g., nucleic acid, that enters the target cell in the presence of the agent is at least about 2-fold, such as at least about 5-fold, including at least about 10-fold or more, greater than that observed in a control situation, e.g., where the agent is not used. The amount of transfer can be determined using any convenient protocol, including but not limited to, the methods described in U.S. Patent No. 6,677,445. A variety of different types of transfection agents are known, including but not limited to: lipid-based transfection agents, polypeptide-based transfections, e.g., polylysine; dendrimer based transfection agents; etc. Transfection agents are described in, for example, U.S. Patent Nos. 6,756,054; 6,753,424; 6,733,777; 6,528,312; 6,479,464; 6,475,994; 6,372,499; 6,320,030; 6,303,300; 6,187,760; 6,187,588; 6,171,612; 6,133,026; 6,124,270; 6,107,286; 6,096,716; 6,074,667; 5,985,573; 5,948,878; 5,851,818; 5,843,643; 5,780,053; 5,756,122; 5,733,762; 5,279,833; etc.

"Lipid-based transfection agent" as used herein refers to a transfection agent which includes a lipid (i.e., a fatty, waxy or oily compound that is characteristically insoluble in water but readily soluble in organic solvents, and contains carbon, hydrogen and oxygen) or liposome component. Liposomes are small vesicles formed from bilayers of amphipathic molecules such as dipalmitoylphospatidyl ethanolamine, dioleylphosphatidyl ethanolamine, stearate salts, cholesterol, and the like. Liposomes may have a single bilayer or multiple bilayers, and may be prepared by a variety of different methods. Other lipids may be used for lipid transfection without forming liposomes, such as for example LIPOFECTIN^{™} transfection agent (also known as DOTMA, N[1-(2,3-dioleyloxy)-propyl]-N,N,N-trimethylammonium chloride, Life Technologies, Inc.).

Specific commercially available lipid-based transfection agents include, but are not limited to: LIPOFECTIN^{™} transfection reagent, OLIGOFECTIN^{™} transfection reagent, DMRIE-C^{™} transfection reagent, LIPOFECTAMINE^{™} transfection reagent, LIPOFECTAMINE PLUS^{™} transfection reagent; LIPOFECTAMINE^{™} 2000 transfection reagent; 1,2-dioleoyl-sn-glycero-3-trimethylammonium-propane (DOTAP); TRANSFAST^{™} transfection reagent; EFFECTENE^{™} transfection reagent; FUGENE 6^{™} transfection reagent; and the like.

As use herein, the phrase "transfection agent mediated cytotoxic response" refers to a non-specific response of a cell to contact with a transfection agent, i.e., a non-specific cytotoxic response that is caused by a transfection agent. Transfection agent mediated cytotoxic responses do not necessarily result in loss of cell viability or a reduction in cell proliferation but may represent a continuum of changes in cell phenotype and a change in the gene expression pattern. As such, in certain embodiments, the transfection agent mediated cytotoxic response is not accompanied by cell death. Depending on the particular transfection agent, the particular transfection agent mediated cytotoxic response may vary, and be accompanied by a variety of different phenotypic characteristics. Phenotypic characteristics include, but are not limited to: changes in the up and down regulation of genes, e.g., in the areas of immune, inflammatory, and stress responses. In certain embodiments, the cytotoxic response is characterized by the presence of a cytotoxic marker gene expression profile (see below) having at least one of, including at least two or more, such as 5 or more of, 10 or more of, 15 or more, 20 or more of 25 or more, including the entire set of genes listed in Table 1, where the genes are up regulated at least about 2-fold relative to a lipid-transfected control that did contain agent of delivery. (For example, an assay may be run where sample 1 would be lipid alone and sample 2 would be lipid+nucleic acid or a different transfection of lipid alone. The RNA is islated, labeled, and hybridized to a microarray. If the ratio of the signal for sample 1 to the signal of sample 2 is > 2-fold for the gene set in Table 1 or a subset of the list, then the lipid alone has induced a cytotoxic response. This result tells the operator that gene expression changes are heavily influenced by the lipid transfection and not the agent that was delivered.)

As used herein, a "cytotoxic marker" refers to any cellular response whose presence is indicative of or associated with the non-specific cytotoxic response from lipid transfection. Cytotoxic markers areany cellular response or collection of cellular responses, as determined either qualitatively or quantitatively, that are associated with a transfection agent mediated cytotoxic response. Cytotoxic markers may vary, where representative cytotoxic markers include, but are not limited to: nucleic acid transcripts, e.g., mRNAs, of the one or more genes that are up or down regulated in response to the lipid transfection, polypeptides, e.g., proteins, where the proteins/polypeptides are expression products of the one or more genes of interest, as well as the products of posttranscriptional, posttranslational modifications/processing events of such elements that occur as cytotoxic responses.

A cytotoxic marker profile is one or more data values that represents the presence of the marker, either qualitatively or quantitatively. The cytotoxic marker profile may include a value for a single cytotoxic marker, or it may include the values of a plurality of markers, e.g., two or more markers, such as 5 or more, 10 or more, 15 or more, 25 or more, etc., markers.

In certain embodiments, the cytotoxic marker is an expression product of a cytotoxic marker gene. A cytotoxic marker gene is a gene that expresses a product, such as the compounds listed above, where the presence and/or amount of the expressed product is associated with the cytotoxic response of interest. The term "expression" with respect to a gene sequence refers to the production of a protein or nucleic acid sequence in a cell. The term includes transcription into an RNA product, post-transcriptional modification and/or translation to a protein product or polypeptide from a gene encoding that product, as well as possible post-translational modifications.

In certain embodiments, the cytotoxic marker profile is a cytotoxic marker gene expression profile of one or more cytotoxic marker gene. A cytotoxic marker gene is a gene that expresses a product, such as the compounds listed above, where the presence and/or amount of the expressed product is associated with the cytotoxic response of interest. A gene expression profile is a set of expression data for one or more genes, where the expression profile may be a nucleic acid or polypeptide expression profile, or combination thereof. As used herein, "changes in gene expression" or "differences in gene expression" refer to a measurable change or difference in the level of protein and/or mRNA product from a target genomic domain or region in a cell over time, between a control and experimental cell, or between distinct cells or populations of cells. Changes or differences in gene expression can be measured by examination of the outward properties of the cell or organism (e.g., phenotype) or by biochemical techniques that include, but are not limited to, RNA solution hybridization, nuclease protection, Northern hybridization, reverse transcription, gene expression monitoring with a microarray, antibody binding, enzyme linked immunosorbent assay (ELISA), Western blotting, radioimmunoassay (RIA), other immunoassays, and fluorescence activated cell analysis (FACS). As used herein a "measurable change" refers to a change in magnitude of a given metric that is at least about 2-fold, such as at least about 5-fold including at least about 10-fold different from as suitable control or reference value.

The terms "reference" and "control" are used interchangebly to refer to a known value or set of known values against which an observed value may be compared. As used herein, known means that the value represents an understood parameter, e.g., a level of expression of a cytotoxic marker gene is the absence of contact with a transfection agent.

"Cells," "target cells," "host cells" or "recombinant host cells" are terms used interchangeably herein. It is understood that such terms refer not only to the particular subject cell but to the progeny or potential progeny of such a cell. Because certain modifications may occur in succeeding generations of cells due to either mutation or environmental influences, such progeny may not, in fact, be identical to the parent cell, but are still included within the scope of the term as used herein. As used herein, "transformed cells" refers to cells that have spontaneously converted to a state of unrestrained growth, e.g., they have acquired the ability to grow through an indefinite number of divisions in culture. Transformed cells may be characterized by such terms as neoplastic, anaplastic and/or hyperplastic, with respect to their loss of growth control. As used herein, "immortalized cells" refers to cells that have been altered via chemical, genetic, and/or recombinant means such that the cells have the ability to grow through an indefinite number of divisions in culture. As used herein, "primary cells" refers to cells derived from a tissue of an organism wherein said cells have not been altered such that they can grow through an indefinite number of divisions in culture.

As used herein, a "reporter gene construct" is a nucleic acid that includes a "reporter gene" operatively linked to at least one transcriptional regulatory sequence (TRE). Reporter gene constructs can be episomal plasmid vectors or integrated into the host cell genome. Transcription of the reporter gene is controlled by the TRE to which they are linked. Reporter genes typically encode proteins whose presence or activity can be readily measured. Examples include, but are not limited to, alkaline phosphatase (AP), beta galactosidase (LacZ), beta glucoronidase (GUS), chloramphenicol acetyltransferase (CAT), green fluorescent protein (GFP), horseradish peroxidase (HRP), and luciferase (Luc). Exemplary transcriptional control sequences are promoter sequences but can also include known or suspected transcription factor binding sites derived from promoters or designed to mimic such elements.

As used herein, the phrase transfection agent complexed with a cargo agent, also referred to as a transfection agent cargo complex, refers to any composition that includes a transfection agent bound to a cargo agent, where the nature of the bond may be covalent or non-covalent, but in certain embodiments of interest is non-covalent.

A "cargo agent" (also referred to herein simply as "cargo") may be any of a variety of different types of agents, and in representative embodiments is a biopolymer. A "biopolymer" is a polymer of one or more types of repeating units. Biopolymers are typically found in biological systems and particularly include polysaccharides (such as carbohydrates), and peptides (which term is used to include polypeptides, and proteins whether or not attached to a polysaccharide) and polynucleotides as well as their analogs such as those compounds composed of or containing amino acid analogs or non-amino acid groups, or nucleotide analogs or non-nucleotide groups. As such, this term includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions. Polynucleotides include single or multiple stranded configurations, where one or more of the strands may or may not be completely aligned with another. Specifically, a "biopolymer" includes DNA (including cDNA), RNA and oligonucleotides, regardless of the source.

The term "nucleic acid" includes DNA, RNA (double-stranded or single stranded), analogs (e.g., PNA or LNA molecules) and derivatives thereof. The terms "ribonucleic acid" and "RNA" as used herein mean a polymer composed of ribonucleotides. The terms "deoxyribonucleic acid" and "DNA" as used herein mean a polymer composed of deoxyribonucleotides. The term "mRNA" means messenger RNA. An "oligonucleotide" generally refers to a nucleotide multimer of about 10 to 100 nucleotides in length, while a "polynucleotide" includes a nucleotide multimer having any number of nucleotides.

The terms "protein" and "polypeptide" used in this application are interchangeable. "Polypeptide" refers to a polymer of amino acids (amino acid sequence) and does not refer to a specific length of the molecule. Thus peptides and oligopeptides are included within the definition of polypeptide. This term does also refer to or include post-translational modifications of the polypeptide, for example, glycosylations, acetylations, phosphorylation and the like. Included within the definition are, for example, polypeptides containing one or more analogs of an amino acid, polypeptides with substituted linkages, as well as other modifications known in the art, both naturally occurring and non-naturally occurring.

An "RNAi agent" is an agent that reduces the expression of a target gene by a mechanism called RNA interference (RNAi) or posttranscriptional gene silencing (PTGS). This mechanism is induced in cells by double-stranded RNA that is homologous to the target gene (silencing trigger) and is mediated by RNA-induced silencing complex (RISC), a sequence-specific, multicomponent nuclease that destroys messenger RNAs homologous to the silencing trigger. RNAi agents can be small ribonucleic acid molecules, e.g., oligoribonucleotides, that are present in duplex structures, e.g., two distinct oligoribonucleotides hybridized to each other or a single ribooligonucleotide that assumes a small hairpin formation to produce a duplex structure. In addition, RNAi agents may be a transcriptional template of the interfering ribonucleic acid. In these embodiments, the transcriptional template is typically a DNA that encodes the interfering ribonucleic acid. The DNA may be present in a vector, where a variety of different vectors are known in the art, e.g., a plasmid vector, a viral vector, etc. See e.g., U.S. Patent Nos. 6,573,099 and 6,506,559.

A cytotoxic marker probe is any agent that specifically binds to a cytotoxic marker. The probe may be a variety of different specific binding agents that, by virtue of their composition and/or configuration, bind specifically to a second agent, where representative binding agents include nulceic acids and polypetides, e.g., antibodies or binding fragments thereof.

A gene specific primer is a nucleic acid of sufficient length to serve as a primer for a template dependent primer extension reaction and has a sequence that hybridizes under stringent conditions to a target sequence that is to serve as a template in a template dependent primer extension reaction.

A "biomonomer" references a single unit, which can be linked with the same or other biomonomers to form a biopolymer (for example, a single amino acid or nucleotide with two linking groups one or both of which may have removable protecting groups). A biomonomer fluid or biopolymer fluid reference a liquid containing either a biomonomer or biopolymer, respectively (typically in solution).

A "nucleotide" refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides.

A chemical "array", unless a contrary intention appears, includes any one, two or three-dimensional arrangement of addressable regions bearing a particular chemical moiety or moieties (for example, biopolymers such as polynucleotide sequences) associated with that region. For example, each region may extend into a third dimension in the case where the substrate is porous while not having any substantial third dimension measurement (thickness) in the case where the substrate is non-porous. An array is "addressable" in that it has multiple regions (sometimes referenced as "features" or "spots" of the array) of different moieties (for example, different polynucleotide sequences) such that a region at a particular predetermined location (an "address") on the array will detect a particular target or class of targets (although a feature may incidentally detect non-targets of that feature). The target for which each feature is specific is, in representative embodiments, known. An array feature is generally homogenous in composition and concentration and the features may be separated by intervening spaces (although arrays without such separation can be fabricated).

In the case of an array, the "target" will be referenced as a moiety in a mobile phase (typically fluid), such as a sample, to be detected by probes (e.g., cytotoxic probes) which are bound to the substrate at the various regions. However, either of the "target" or "target probes" may be the one which is to be detected by the other (thus, either one could be an unknown mixture of polynucleotides to be detected by binding with the other). "Addressable set of probes" and analogous terms refers to the multiple regions of different moieties supported by or intended to be supported by the array surface.

An "array layout" or "array characteristics", refers to one or more physical, chemical or biological characteristics of the array, such as positioning of some or all the features within the array and on a substrate, one or more feature dimensions, or some indication of an identity or function (for example, chemical or biological) of a moiety at a given location, or how the array should be handled (for example, conditions under which the array is exposed to a sample, or array reading specifications or controls following sample exposure).

"Hybridizing" and "binding", with respect to polynucleotides, are used interchangeably.

A "plastic" is any synthetic organic polymer of high molecular weight (for example at least 1,000 grams/mole, or even at least 10,000 or 100,000 grams/mole.

"Flexible" with reference to a substrate or substrate web (including a housing or one or more housing component such as a housing base and/or cover), references that the substrate can be bent 180 degrees around a roller of less than 1.25 cm in radius. The substrate can be so bent and straightened repeatedly in either direction at least 100 times without failure (for example, cracking) or plastic deformation. This bending must be within the elastic limits of the material. The foregoing test for flexibility is performed at a temperature of 20 °C. "Rigid" refers to a substrate (including a housing or one or more housing component such as a housing base and/or cover) which is not flexible, and is constructed such that a segment about 2.5 by 7.5 cm retains its shape and cannot be bent along any direction more than 60 degrees (and often not more than 40, 20, 10, or 5 degrees) without breaking.

When one item is indicated as being "remote" from another, this descriptor indicates that the two items are at least in different buildings, and may be at least one mile, ten miles, or at least one hundred miles apart. When different items are indicated as being "local" to each other they are not remote from one another (for example, they can be in the same building or the same room of a building). "Communicating", "transmitting" and the like, of information reference conveying data representing information as electrical or optical signals over a suitable communication channel (for example, a private or public network, wired, optical fiber, wireless radio or satellite, or otherwise). Any communication or transmission can be between devices which are local or remote from one another. "Forwarding" an item refers to any means of getting that item from one location to the next, whether by physically transporting that item or using other known methods (where that is possible) and includes, at least in the case of data, physically transporting a medium carrying the data or communicating the data over a communication channel (including electrical, optical, or wireless). "Receiving" something means it is obtained by any possible means, such as delivery of a physical item (for example, an array or array carrying package). When information is received it may be obtained as data as a result of a transmission (such as by electrical or optical signals over any communication channel of a type mentioned herein), or it may be obtained as electrical or optical signals from reading some other medium (such as a magnetic, optical, or solid state storage device) carrying the information. However, when information is received from a communication it is received as a result of a transmission of that information from elsewhere (local or remote).

When two items are "associated" with one another they are provided in such a way that it is apparent one is related to the other such as where one references the other. For example, an array identifier can be associated with an array by being on the array assembly (such as on the substrate or a housing) that carries the array or on or in a package or kit carrying the array assembly. Items of data are "linked" to one another in a memory when a same data input (for example, filename or directory name or search term) retrieves those items (in a same file or not) or an input of one or more of the linked items retrieves one or more of the others. In particular, when an array layout is "linked" with an identifier for that array, then an input of the identifier into a processor which accesses a memory carrying the linked array layout retrieves the array layout for that array.

A "computer", "processor" or "processing unit" are used interchangeably and each references any hardware or hardware/software combination which can control components as required to execute recited steps. For example a computer, processor, or processor unit includes a general purpose digital microprocessor suitably programmed to perform all of the steps required of it, or any hardware or hardware/software combination which will perform those or equivalent steps. Programming may be accomplished, for example, from a computer readable medium carrying necessary program code (such as a portable storage medium) or by communication from a remote location (such as through a communication channel).

A "memory" or "memory unit" refers to any device which can store information for retrieval as signals by a processor, and may include magnetic or optical devices (such as a hard disk, floppy disk, CD, or DVD), or solid state memory devices (such as volatile or non-volatile RAM). A memory or memory unit may have more than one physical memory device of the same or different types (for example, a memory may have multiple memory devices such as multiple hard drives or multiple solid state memory devices or some combination of hard drives and solid state memory devices).

An array "assembly" includes a substrate and at least one chemical array on a surface thereof. Array assemblies may include one or more chemical arrays present on a surface of a device that includes a pedestal supporting a plurality of prongs, e.g., one or more chemical arrays present on a surface of one or more prongs of such a device. An assembly may include other features (such as a housing with a chamber from which the substrate sections can be removed). "Array unit" may be used interchangeably with "array assembly".

"Reading" signal data from an array refers to the detection of the signal data (such as by a detector) from the array. This data may be saved in a memory (whether for relatively short or longer terms).

A "package" is one or more items (such as an array assembly optionally with other items) all held together (such as by a common wrapping or protective cover or binding). Normally the common wrapping will also be a protective cover (such as a common wrapping or box) which will provide additional protection to items contained in the package from exposure to the external environment. In the case of just a single array assembly a package may be that array assembly with some protective covering over the array assembly (which protective cover may or may not be an additional part of the array unit itself).

It will also be appreciated that throughout the present application, that words such as "cover", "base" "front", "back", "top", "upper", and "lower" are used in a relative sense only.

"May" refers to optionally.

When two or more items (for example, elements or processes) are referenced by an alternative "or", this indicates that either could be present separately or any combination of them could be present together except where the presence of one necessarily excludes the other or others.

The term "stringent assay conditions" as used herein refers to conditions that are compatible to produce binding pairs of nucleic acids, e.g., surface bound and solution phase nucleic acids, of sufficient complementarity to provide for the desired level of specificity in the assay while being less compatible to the formation of binding pairs between binding members of insufficient complementarity to provide for the desired specificity. Stringent assay conditions are the summation or combination (totality) of both hybridization and wash conditions.

"Stringent hybridization conditions" and "stringent hybridization wash conditions" in the context of nucleic acid hybridization (e.g., as in array, Southern or Northern hybridizations) are sequence dependent, and are different under different experimental parameters. Stringent hybridization conditions that can be used to identify nucleic acids within the scope of the invention can include, e.g., hybridization in a buffer comprising 50% formamide, 5×SSC, and 1% SDS at 42°C, or hybridization in a buffer comprising 5×SSC and 1% SDS at 65°C, both with a wash of 0.2xSSC and 0.1 % SDS at 65°C. Exemplary stringent hybridization conditions can also include a hybridization in a buffer of 40% formamide, 1 M NaCl, and 1% SDS at 37°C, and a wash in 1×SSC at 45°C. Alternatively, hybridization to filter-bound DNA in 0.5 M NaHPO₄, 7% sodium dodecyl sulfate (SDS), 1 mM EDTA at 65°C, and washing in 0.1 ×SSC/0.1 % SDS at 68°C can be employed. Yet additional stringent hybridization conditions include hybridization at 60°C or higher and 3xSSC (450 mM sodium chloride/45 mM sodium citrate) or incubation at 42°C in a solution containing 30% formamide, 1 M NaCl, 0.5% sodium sarcosine, 50 mM MES, pH 6.5. Those of ordinary skill will readily recognize that alternative but comparable hybridization and wash conditions can be utilized to provide conditions of similar stringency.

In certain embodiments, the stringency of the wash conditions that set forth the conditions which determine whether a nucleic acid is specifically hybridized to a surface bound nucleic acid. Wash conditions used to identify nucleic acids may include, e.g.: a salt concentration of about 0.02 molar at pH 7 and a temperature of at least about 50°C or about 55°C to about 60°C; or, a salt concentration of about 0.15 M NaCl at 72°C for about 15 minutes; or, a salt concentration of about 0.2xSSC at a temperature of at least about 50°C or about 55°C to about 60°C for about 15 to about 20 minutes; or, the hybridization complex is washed twice with a solution with a salt concentration of about 2xSSC containing 0.1 % SDS at room temperature for 15 minutes and then washed twice by 0.1 xSSC containing 0.1 % SDS at 68°C for 15 minutes; or, equivalent conditions. Stringent conditions for washing can also be, e.g., 0.2xSSC/0.1 % SDS at 42°C.

A specific example of stringent assay conditions is rotating hybridization at 65°C in a salt based hybridization buffer with a total monovalent cation concentration of 1.5 M (e.g., as described in U.S. Patent Application No. 09/655,482 filed on September 5, 2000, the disclosure of which is herein incorporated by reference) followed by washes of 0.5xSSC and 0.1 xSSC at room temperature.

Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may also be employed, as appropriate.

The term "assessing" and "evaluating" are used interchangeably to refer to any form of measurement, and includes determining if an element is present or not. The terms "determining," "measuring," "assessing," and "assaying" are used interchangeably and include both quantitative and qualitative determinations. Assessing may be relative or absolute. "Assessing the presence of" includes determining the amount of something present, as well as determining whether it is present or absent.

Methods and compositions for determining cytotoxicity of a transfection agent are provided. In practising the subject methods, a cell is contacted with a transfection agent. Following contact, a cytotoxic marker profile is evaluated to determine whether the transfection agent has mediated a cytotoxic response in the cell. Also provided are compositions, reagents and kits for practicing the subject methods. The subject methods and compositions find use in a variety of different applications.

Before preferred embodiments of the present invention are described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It must be noted that as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

### METHODS

In one aspect, the subject invention provides methods for determining whether a transfection agent has induced a cytotoxic response in a target cell following contact of the cell with the agent. In one aspect, a cell is first contacted with a transfection agent (e.g., such as a lipid-based transfection agent, where the agent may or may not be complexed with a cargo (e.g., a nucleic acid)). Following contact, the presence of at least one cytotoxic marker is evaluated at one or more times to obtain a cytotoxic marker profile. The expression data is then employed to determine whether a transfection agent-mediated non-specific cytotoxicity response has occurred in the cell. Each of these steps of the invention is now described separately in greater detail.

### Cell Contact with a Transfection Agent

As indicated above, the first step in the subject methods is to contact a cell with a transfection agent. As defined above, a "transfection agent" is any of a class of chemical compounds that find use in increasing the efficiency of nucleic acid transfer into a target cell. An agent is considered to increase efficiency of nucleic acid transfer into a target cell, and therefore considered to be a transfection agent, when the amount of nucleic acid that enters the a target cell in the presence of the agent is at least about 2-fold, such as at least about 5-fold, including at least about 10-fold, greater than observed in a control situation, e.g., where the agent is not used. The amount of nucleic acid transfer can be determined using any convenient protocol, including but not limited to, the methods described in U.S. Patent No. 6,677,445. A variety of different types of transfection agents are known, including but not limited to: lipid-based transfection agents, polypeptide based transfections, e.g., polylysine; dendrimer based transfection agents; etc. Transfection agents are described in, for example, U.S. Patent Nos. 6,756,054; 6,753,424; 6,733,777; 6,528,312; 6,479,464; 6,475,994; 6,372,499; 6,320,030; 6,303,300; 6,187,760; 6,187,588; 6,171,612; 6,133,026; 6,124,270; 6,107,286; 6,096,716; 6,074,667; 5,985,573; 5,948,878; 5,851,818; 5,843,643; 5,780,053; 5,756,122; 5,733,762; 5,279,833; etc.

"Lipid-based transfection agent" as used herein refers to a transfection agent which includes a lipid (i.e., a fatty, waxy or oily compound that is characteristically insoluble in water but readily soluble in organic solvents, and contains carbon, hydrogen and oxygen) or liposome component. Liposomes are small vesicles formed from bilayers of amphipathic molecules such as dipalmitoylphospatidyl ethanolamine, dioleylphosphatidyl ethanolamine, stearate salts, cholesterol, and the like. Liposomes may have a single bilayer or multiple bilayers, and may be prepared by a variety of different methods. Other lipids may be used for lipid transfection without forming liposomes, such as for example LIPOFECTIN^{™} transfection agent (also known as DOTMA, N[1-(2,3-dioleyloxy)-propyl]-N,N,N-trimethylammonium chloride, Life Technologies, Inc.).

Specific commercially available lipid based transfection agents include, but are not limited to: may include but not limited to LIPOFECTIN^{™} transfection reagent, OLIGOFECTIN^{™} transfection reagent, DMRIE-C^{™} transfection reagent, LIPOFECTAMINE^{™} transfection reagent, LIPOFECTAMINE PLUS^{™} transfection reagent; LIPOFECTAMINE^{™} 2000 transfection reagent; 1,2-dioleoyl-sn-glycero-3-trimethylammonium-propane (DOTAP); TRANSFAST^{™} transfection reagent; EFFECTENE^{™} transfection reagent; FUGENE 6^{™} transfection reagent; and the like. The transfection agent can be contacted with the target cell either alone or in combination with other lipid-based transfection reagents or with other chemical compounds, e.g., such as agents that function to enhance the efficiency of nucleic acid uptake by target cells. Examples of such agents include, but are not limited to, cell membrane components, e.g., cholesterol (da Cruz et al., 2004, Experimental Neurology 187:65), zwitterionic lipids, e.g., dioleoylphosphatidylethanolamine (DOPE) (Ciani et al. 2004, Biochimica et Biophysica Acta 1664:70), polypeptides (Torchilin et al., 2003, PNAS 100:1972), proteins (Duzgunes, 2003, Current Medicinal Chemistry 10:1213), and heavy oils (Yoo et al., 2004, Journal of Controlled Release 98:179).

In certain embodiments, the transfection agent that is contacted with a target cell is one that is complexed with a "cargo" agent, e.g., a nucleic acid. In some embodiments, the cargo nucleic acid is a DNA molecule. The DNA molecule can be a plasmid vector, e.g., that is designed to drive the expression of a particular gene product (or protein). Alternatively, the plasmid vector can be designed to monitor a particular cellular process. One such vector is a reporter gene vector which contains a specific promoter, or transcription response element, functionally linked to a gene whose expression can be measured. The DNA vector may also be designed to express a particular functional RNA molecule, including ribozymes, antisense RNA molecules, RNAi agents, or tRNAs. The DNA may also be an integrating DNA vector, which is designed to integrate into the host cell genome, thereby maintaining its presence in the daughter cells after cell division. Integrating vectors can vary widely depending on the desired outcome. They can be very large, sometimes in the megabase range, and can be either in a linear or closed circular configuration. In still other embodiments, the nucleic acid transfected into the target cells is a DNA oligonucleotide. DNA oligonucleotides can be antisense oligonucleotides which have a sequence that is complimentary to a specific mRNA and can therefore form a DNA:RNA heteroduplex which either leads to mRNA destruction or prevents efficient translation of the mRNA in the target cell. Also, included within the scope of the invention are nucleic acid analogs (e.g., PNA or LNA molecules) and modified forms thereof.

In some embodiments, the nucleic acid transfected into the target cells is an RNA molecule. In certain of these embodiments, the RNA molecule is an mRNA which can be translated into a specific protein. In other of these embodiments, the RNA molecule is an RNAi agent that can mediate the degradation of a specific mRNA species thereby preventing its translation. In still other of these embodiments, the RNA molecule is an anti-sense RNA which binds to a specific mRNA in the target cell thereby inhibiting its translation.

The transfection agent/cargo complexes may be prepared using any convenient protocol. In certain representative embodiments, a lipid-based transfection agent is combined with the nucleic acid, which may be present in an aqueous solution, *in vitro* such that the charge:charge ratio (lipid:nucleic acid) is within a specified range, such as between about 1:1 and about 10:1, including between about 1:1 and about 3:1. The concentration of nucleic acid in the mixture may vary depending on the type of cell being transfected, but in representative embodiments is in a range of about 1µg/ml to about 20µg/ml. The total amount of nucleic acid applied to cells also varies depending on the target cell type and may range from about 2×10⁻⁶ µg nucleic acid/cell to about 100×10⁻⁶ µg nucleic acid/cell. The components of the complex (transfection agent, cargo,) may be mixed, as desired, to ensure complete intermingling of the lipid-based transfection agent and nucleic acid, and, in representative embodiments, may be incubated at from about 20°C to about 27°C for between about 1 minute to about 30 minutes to allow formation of the desired complex.

In general, the transfection agent, or complex thereof, may be contacted with a variety of different target cells. In some embodiments, the target cells are from species that are "mammals" or "mammalian," where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In certain of these embodiments, the target cells are propagated in *in vitro* cultures. These cells can be transformed cells, immortalized cells, or primary cells.

Contact of the transfection agent and the target cell is conducted in a manner that allows and/or promotes their direct interaction while maintaining the integrity of both. As such, the transfection agent is contacted with the target cell under conditions that allow and/or promote contact. In certain embodiments, contact of the agent and target cell occurs in a fluid-contacting medium. In representative embodiments in which the target cell is a cell that is propagated in *in vitro* tissue culture, the standard growth medium in which the cell is present is acceptable for use as a contacting medium. In certain representative embodiments where serum and/or antibiotics have detrimental effects, such may be excluded from the contacting media as desired. Exemplary conditions and medium for contacting are described U.S. Patent Nos. 6,756,054; 6,753,424; 6,733,777; 6,528,312; 6,479,464; 6,475,994; 6,372,499; 6,320,030; 6,303,300; 6,187,760; 6,187,588; 6,171,612; 6,133,026; 6,124,270; 6,107,286; 6,096,716; 6,074,667; 5,985,573; 5,948,878; 5,851,818; 5,843,643; 5,780,053; 5,756,122; 5,733,762; 5,279,833; etc.

The contacting step is typically done in conditions that support growth of the target cells. For mammalian cell culture, representative conditions are 37°C, from about 5% to about 7% CO₂, and a humidified atmosphere. The contacting step can last for from about 30 minutes to about 16 hours, including from about 3 hours to about 6 hours. In some embodiments, the initial contacting medium is removed from the cells and replaced with fresh cell culture media after the contacting step and the cells are maintained in culture until further analysis. In other embodiments, the contacting solution is left on the cells until analysis. Where desired, the lipid-based transfection agent may be added directly to the cells in growth medium and dispersed gently to maximize contact with between the cells and the lipoplexes.

### Cytotoxic Marker Evaluation

Following contact of the transfection agent, as described above, a cytotoxic marker profile is obtained for the cell. In this step, the target cell is evaluated for the presence of one or more cytotoxic markers, e.g., qualititative or quantitatively, where the one or more cytotoxic markers whose presence is evaluated generally includes at least one marker whose presence has been associated with transfection agent-mediated non-specific cytotoxicity. Cytotoxic marker evaluation is, in certain embodiments, performed at any convenient time following contact of the cell with the agent, so long as the time period between cell contact and evaluation is sufficient for the transfection agent to have caused a cytotoxic response if such is to occur. In representative embodiments, this time period is at least about 0.1 hours, such as at least about 0.25 hours, such as at least about 0.5 hours, such as at least about 1 hour, such as at least about 5 hours, and may be as great as about 24 hours or greater, such as at greater than about 72 hours.

Accordingly, in practicing the subject methods, the transfection-agent contacted cell is assayed to obtain a cytotoxic marker profile, for one or more cytotoxic markers, where the term cytotoxic marker profile is used broadly to include a quantitative or qualititative profile of cytotoxic markers, where the markers may be as described above, such as nucleic acid transcripts, e.g., mRNAs, of the one or more genes of interest, or a proteomic expression profile, e.g., an expression profile of one or more different proteins, where the proteins/polypeptides are expression products of the one or more genes of interest, as well as the products of posttranscriptional, posttranslational modifications/processing events of such that occur as cytotoxic responses.

In certain embodiments, the cytotoxic marker profile that is obtained is an expression profile, and more particularly an expression profile of one or more cytotoxic marker genes. In these embodiments, the transfection-agent contacted cell is assayed to obtain a cytotoxic gene expression profile for one or more cytotoxic marker genes, where the term cytotoxic marker gene profile is used broadly to include a quantitative or qualititative profile of cytotoxic marker gene expression products, where the markers may be as described above, such as nucleic acid transcripts, e.g., mRNAs, of the one or more genes of interest, or a proteomic expression profile, e.g., an expression profile of one or more different proteins, where the proteins/polypeptides are expression products of the one or more genes of interest.

As such, in certain embodiments the presence of only one cytotoxic marker is evaluated. In yet other embodiments, the presence of two or more, e.g., about 5 or more, about 10 or more, about 15 or more, about 25 or more, about 50 or more, about 100 or more, about 200 or more, etc., cytotoxic markers are evaluated.

In generating the cytotoxic maker profile, in certain embodiments, a sample is assayed to generate a profile that includes data for at least one cytotoxic marker, usually a plurality of cytotoxic markers, where by plurality is meant at least two different cytotoxic makers, and often at least about 5, typically at least about 10 and more usually at least about 20 different cytotoxic markers or more, such as 50 or more, 100 or more, etc.

As indicated above, the evaluation of a given cytotoxic marker may be qualitative or quantitative. As such, where detection is qualitative, the methods provide a reading or evaluation, e.g., assessment, of whether or not a given cytotoxic marker is present in the sample being assayed. In yet other embodiments, the actual amount or relative abundance of the cytotoxic marker of interest is determined (e.g., in quantitative assays). In such embodiments, the quantitative detection may be absolute or, if the method is a method of detecting two or more different cytotoxic markers, e.g., products of different genes, in a sample, relative. As such, the term "quantifying" when used in the context of quantifying a cytotoxic marker in a sample can refer to absolute or to relative quantification. Absolute quantification may be accomplished by inclusion of known concentration(s) of one or more controls and referencing the detected level of the marker with the known controls (e.g., through generation of a standard curve). Alternatively, relative quantification can be accomplished by comparison of detected levels or amounts between two or more different cytotoxic markers to provide a relative quantification of each of the two or more different cytotoxic markers, e.g., relative to each other.

As reviewed above, cytotoxic markers of interest include transcripts/proteins that are differentially expressed or present at different levels in a cell depending on whether the transfection agent has caused a non-specific cytotoxic response in the cell (e.g., unrelated to the sequence properties of a nucleic acid). In one aspect, a given gene is considered to be expressed at a "different level" if, when compared to a control, e.g., by using the representative protocols provided in the Experimental Section below, the obtained expression signal or value derived therefrom is at least about 2-fold, such as at least about 5-fold including at least about 10-fold different in magnitude from the control or reference value. In another aspect, a given gene is considered to be expressed at a "different level" if, when compared to a control, the obtained expression signal or value derived therefrom is at least about 10%, at least about 20%, at least about 30% or greater, different in magnitude from the control or reference value. In still another aspect, a given gene is considered to be expressed at a "different level" if, when compared to a control, the obtained expression signal or value derived therefore is statistically significantly different from the control or reference value (p < 0.05). Representative genes/proteins of interest in certain embodiments include, but are not limited to, the genes/proteins provided in Table 1, infra (See the Experimental Section).

In certain embodiments, at least one of the genes in the prepared expression profile is from Table 1, where the expression profile may include expression data for a plurality of such genes, where the term plurality means at least 2, such as 5, 10, 20, 50, 75 or more of, including all of, the genes/proteins listed in Table 1. The number of different genes whose expression profiles are evaluated may vary, but may be at least 2, and in some embodiments ranges from 2 to about 100 or more, sometimes from 3 to about 75 or more, including from about 4 to about 70 or more.

In certain representative embodiments, the expression of at least one of IF127 and MX1 is evaluated (see Table 1), where in certain embodiments the expression profile of at least both of these genes is evaluated.

In certain embodiments, the expression profile obtained is a genomic (i.e. nucleic acid) expression profile, where the amount (i.e. level) of one or more nucleic acids in the sample is determined, e.g., the transcript of the gene of interest or a copy thereof (e.g., a cDNA). In these embodiments, target cell-derived sample is assayed. The sample may comprise a cell lysate, a fraction thereof, or purified or partially purified nucleic acids. The nucleic acid sample includes a plurality or population of distinct nucleic acids that includes the expression information of the phenotype-determinative genes of interest for the cell or tissue being profiled. The nucleic acid may include RNA or DNA nucleic acids, e.g., mRNA, cRNA, cDNA etc., so long as the sample retains the expression information of the target cell from which it is obtained. The sample may be prepared in a number of different ways, as is known in the art, e.g., by mRNA isolation from a cell, where the isolated mRNA is used as is, amplified, employed to prepare cDNA, cRNA, etc., as is known in the art.

An expression profile may be generated from the nucleic acid sample using any convenient protocol. While a variety of different manners of generating expression profiles are known, such as those employed in the field of differential gene expression analysis, one representative and convenient type of protocol for generating expression profiles is array-based gene expression profile generation protocol. In such protocols, hybridization assays in which a nucleic acids comprising "probe" sequences are employed. In these assays, a sample of target nucleic acids is first prepared from the initial nucleic acid sample being assayed, where preparation may include labeling of the target nucleic acids with a label, (e.g., such as a member of signal producing system). Following target nucleic acid sample preparation, the sample is contacted with an array comprising probe sequences under hybridization conditions and complexes are formed between target nucleic acids that are complementary to probe sequences attached to the array surface. The presence of complexes is then detected, either qualitatively or quantitatively. Specific hybridization technology which may be practiced to generate the expression profiles employed in the subject methods includes, but is not limited to, the technology described in U.S. Patent Nos.: 6,656,740; 6,613,893; 6,599,693; 6,589,739; 6,587,579; 6,420,180; 6,387,636; 6,309,875; 6,232,072; 6,221,653; and 6,180,351 and the references cited therein.

Alternatively, non-array based methods for quantitating the levels of one or more nucleic acids in a sample may be employed, including quantitative PCR, and the like.

Where the expression profile is a protein expression profile, any convenient protein quantitation protocol may be employed, where the levels of one or more proteins in the assayed sample are determined. Representative methods include, but are not limited to: proteomic arrays (see e.g., 6,475,809), flow cytometry (see e.g., 4,600,302; 4,989,977; and 5,700,692), standard immunoassays (e.g., ELISA assays) (See HARLOW & LANE, USING ANTIBODIES: A LABORATORY MANUAL (1998), etc.

### Use of Cytotoxic Marker Profile to Detect Transfection Agent-Mediated Non-Specific Cytotoxic Response

In one aspect, a cytotoxic marker profile is employed to detect the occurrence of a transfection agent-mediated non-specific cytotoxic event in the target cell being assayed.

In some embodiments, the cytotoxic marker profile is compared with a reference (also referred to herein as a control profile) to determine whether a transfection agent-mediated non-specific cytotoxic response has occurred. The reference of these embodiments can be in the form of a standardized pattern, e.g., of gene expression or levels of expression of certain genes to be used to interpret the profiles of cytotoxic marker(s) in a cell contacted with a transfection agent. The reference profile may be a profile that is obtained from a cell known to have the phenotype of interest, e.g., cytotoxic phenotype, and therefore may be a positive reference profile. In addition, the reference profile may be from a cell known to not have the desired phenotype, e.g., a cell not contacted with a transfection agent, and therefore be a negative reference profile. Both positive and negative references may be used as controls to interpret the cytotoxic marker profiles.

In certain embodiments, the profile of cytotoxic markers is compared to a single reference profile to obtain information regarding whether the cell being assayed has undergone a transfection agent-mediated cytotoxic response. In yet other embodiments, the obtained expression profile is compared to two or more different reference profiles to obtain more in depth information regarding whether cytotoxicity has been induced in the assayed cell after being contacted with the transfection agent. For example, the obtained profile may be compared to a positive and negative reference profile to obtain information regarding whether the cell has undergone a transfection agent-mediated cytotoxic response.

The comparison of the obtained expression profile and the one or more reference profiles may be performed using any convenient methodology, where a variety of methodologies are known to those of skill in the array art, e.g., by comparing digital images of the expression profiles, by comparing databases of expression data, etc. Patents describing ways of comparing expression profiles include, but are not limited to, U.S. Patent Nos. 6,308,170 and 6,228,575, the disclosures of which are herein incorporated by reference. Methods of comparing expression profiles are also described above.

In some embodiments, the signal obtained from a given feature corresponding to a probe of interest is obtained and, following any desired background, noise or other correction, is compared to a reference value. A difference of at least about 2-fold, such as at least about 5-fold, including at least about 10-fold the magnitude of intensity of the signal, e.g., either greater or less intense than the reference or control value, indicates that a difference exists between the expression of the gene of interest in the test sample as compared to the control. In another aspect, a given gene is considered to be expressed at a "different level" if, when compared to a control, the obtained expression signal or value derived therefrom is at least about 10%, at least about 20%, at least about 30% or greater, different in magnitude from the control or reference value. In still another aspect, a given gene is considered to be expressed at a "different level" if, when compared to a control, the obtained expression signal or value derived therefore is statistically significantly different from the control or reference value (p < 0.05). A more detailed representative protocol for comparing expression levels is provided in the Experimental Section, below.

The comparison step results in information regarding the occurrence of a transfection agent-mediated cytotoxic response in the target cell. By cytotoxic response is meant any non-specific response induced by a transfection agent and not the specific cargo that may be carried thereby, as defined above. In one aspect, the cytotoxic reponse that is identified is a lipid-based transfection reagent cytotoxic response, such as a cytotoxic response associated with inhibition (e.g., cationic amphiphile mediated inhibition) of protein kinase C activity, (see e.g., Aberle et al., Biochemistry (1998) 37: 6533-6540. A target cell is considered to have had a cytotoxic response to a lipid-based transfection agent if it displays at least one of the following characterists: upregulation of the genes listed in Table 1 by greater than 2-fold or upregulation of genes involved in the immune, inflammatory, and stress responses.

Practice of the above method results in the determination of whether a transfection agent mediated cytotoxic response has occurred in cell that has been contacted with the agent.

In certain embodiments, the subject methods further include determination of non-transfection mediated non-specific cytotoxic responses in the target cell. Non-transfection agent mediated non-specific cytotoxic responses are non-specific cytotoxic response that are mediated by an agent other than a transfection agent, e.g., a cargo agent. Representative non-transfection agent mediated non-specific cytotoxicity responses include, but are not limited to: (a) the interferon response to high concentrations of siRNA, as described in Nature Biotechnology 21:635-637; PNAS 101:1892-1897; (b nonspecific, but sequence-dependent effects, of siRNAs acting on other unknown targets (PNAS 101:1982-1897; etc. This type of cytotoxic off-target effect is different from activation of the double-stranded RNA-triggered IFN-associated antiviral pathway.

In these embodiments, the one or more additional non-transfection agent mediated cytotoxic responses of interest may be assayed using any convenient protocol. In those embodiments where the transfection agent mediated cytotoxic response of interest is assayed using an array of cytotoxic marker probes, the array may further include one or more probes for markers of a non-transfection agent mediated non-specific cytotoxic response, where representative markers of such are described in the above references.

The subject methods find use in a variety of different applications where one wishes to evaluate whether a cytotoxic response has been induced in a target cell contacted with a transfection agent.

One particular use of the subject invention is for optimizing a nucleic acid transfection protocol that employs a transfection agent and a specific target cell.
In this application, varying conditions for transfection agent/cargo complex formation, contacting, and culturing are tested. The conditions under which cytotoxic marker(s) are not induced or the fewest number of cytotoxic markers are induced, but transfection of the cargo has occurred, would be considered "optimal".

In one aspect, the invention finds use in providing quality control for experiments in which a transfection agent is used to introduce nucleic acids into a cell. As mentioned above, transfection agents can be used to transfect a number of different nucleic acids including DNA plasmid vectors, DNA oligonucleotides, RNAi agents, etc. In attributing a specific biological outcome to the activity of the specific nucleic acid, it is necessary to determine whether the procedure itself has influenced the outcome.

In one specific embodiment, the present invention finds use as a quality control in experiments that employ transfection agents to test the effect of a library of nucleic acids on a target cell (e.g., RNAi libraries) and to identify -non-specific-transfection agent-mediated cytotoxic response(s).

In one aspect, the invention finds use in analyzing cytotoxic responses induced in the use of transfection agents for the delivery of gene therapy agents in vivo. After contacting target cells with the transfection agent/cargo complexes via the desired route (e.g., injection, through an open surgical field, through a catheter, topically, or using other means know in the art), tissues can be analyzed for changes in gene expression that indicate a non-specific cytotoxic response. In one aspect, the invention thus allows researchers a robust and quantitative tool for determining which transfection agent (including a nucleic acid vector) is optimal for the delivery of gene therapy agents to target cells in vivo.

Although transfection agents are primarily used for the transfection of nucleic acids, transfection agents can also be used to deliver other types of cargo into target cells, including proteins (Ye et al., 2002, Pharmaceutical Research 19:1302). Therefore, the subject invention is not limited to embodiments in which nucleic acids are the cargo.

### KITS

Kits for use in connection with the subject invention are also included within the scope of the invention. Kits generally include cytotoxic probes. In some embodiments, kits also include instructions for performing methods according to one or more different embodiments of the invention.

In one aspect, a subject kit includes an array of cytotoxic probes (e.g., such as nucleic acids) comprising sequences sufficiently complementary to a cytotoxic markers comprising nucleic acids, e.g., such as RNA transcripts whose expression is altered (e.g., upregulated or down-regulated) in a cell undergoing a non-specific transfection agent-mediated cytotoxic response). A variety of different array formats are known in the art, with a wide variety of different probe structures, substrate compositions and attachment technologies. Representative array structures of interest include those described in U.S. Patent Nos.: 6,656,740; 6,613,893; 6,599,693; 6,589,739; 6,587,579; 6,420,180; 6,387,636; 6,309,875; 6,232,072; 6,221,653; and 6,180,351 and the references cited therein. In representative embodiments, the arrays include probes for at least 1 of the genes listed in Table 1. In certain embodiments, the number of genes represented on the array is at least 2, at least 5, at least 10, including all of the genes listed in Table 1. The subject arrays may include additional genes that are not listed in Table 1.

Another type of reagent that is specifically tailored for generating expression profiles of cytotoxic markers which comprise nucleic acids is a collection of primers that is designed to selectively amplify such nucleic acids. As used herein, a collection refers to at least two primers. Primers and methods for using the same are described in U.S. Patent No. 5,994,076. Of particular interest are collections of gene specific primers that have primers for at least 1 of the cytotoxic markers listed in Table 1, including a plurality of these markers, e.g., at least about 2, 5, 10, 15 or more. In certain embodiments, the number of markers from Table 1 that have primers in the collection is at least about 2, at least 5, at least 10, including all of the markers listed in Table 1. In one aspect, the subject primer collections may include primers for additional genes that are not listed in Table 1 and/or other probes suitable for detecting cytotoxic markers (e.g., antibodies, ligands, etc.)

The kits of the subject invention may include the above-described arrays and/or primer collections. The kits may further include one or more additional reagents employed in various methods useful for detecting cytotoxic markers, such as primers for generating target nucleic acids, dNTPs and/or rNTPs, which may be either pre-mixed or separate, one or more uniquely labeled dNTPs and/or rNTPs, such as biotinylated or Cy3 or Cy5 tagged dNTPs, gold or silver particles with different scattering spectra, or other post synthesis labeling reagent, such as chemically active derivatives of fluorescent dyes, enzymes, such as reverse transcriptases, DNA polymerases, RNA polymerases, and the like, various buffer mediums, e.g., hybridization and washing buffers, prefabricated probe arrays, labeled probe purification reagents and components, like spin columns, etc., signal generation and detection reagents, e.g., streptavidin-alkaline phosphatase conjugate, chemifluorescent or chemiluminescent substrate, and the like.

Reagents for evaluating cytotoxic markers can also be designed to evaluate protein levels, and/or protein modifications or processed forms of proteins, e.g., antibodies specific for proteins (and/or their modified or cleaved forms) whose expression is altered (e.g., upregulated or downregulated) in response to non-specific transfection agent-mediated cytotoxicity. Alternatively, or additionally, the activity of such proteins might be measured (e.g., by measuring the conversion of a substrate where the protein is an enzyme). These reagents may be provided in such a form as to allow for western blot analyses, ELISAs, or other protein detection methods known in the art.

In one aspect, the subject kits may also include reagent(s) to perform quality control experiments. These reagents may include a transfection agent (e.g., such as a lipid-based transfection agent) and cells that are known to have a cytotoxic response to the provided lipid-based transfection agent under specific contacting conditions.

In addition to the above components, the subject kits may further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

Additional kits for use in practicing the subject invention may include any collection of reagents compiled to determine whether a non-specific transfection agent-mediated cytotoxic response has occurred in a cell by measuring the changes in the expression of any gene whose expression is associated with lipid-based transfection agent mediated cytotoxicity events, e.g., of the genes in Table 1. These include, but are not limited to, the genomic domain expression detection reagents described above and the supplemental reagents necessary to perform the assay.

The following examples are offered by way of illustration and not by way of limitation.

### I. MATERIALS AND METHODS

### A. Cells and Reagents

HeLa cells were plated at 20K cells per well in 96-well plates and allowed to grow 24 hours at 37 °C and 5% CO₂ before transfection. Transfections were conducted with Lipofectamine 2000^{™} transfection reagent (Invitrogen) according to the manufacturer's instruction. Lipids were delivered both in the presence and absence of siRNA (i.e. "mock" transfection). Cell lysate for microarray analysis was collected 24 hours after transfection and pooled from 12 identically treated sample wells. Total RNA purification was performed using Qiagen's RNeasy columns with on-column DNase digestion. RNA integrity was analyzed with the RNA 6000 Nano LabChip® kit on Agilent's 2100 Bioanalyzer device (Agilent Technologies, Palo Alto CA).

### B. Microarray Procedures

For each sample, 1µg of total RNA was amplified and Cy3- or Cy5-labeled using Agilent's Low Input RNA Fluorescent Linear Amplification Kit (5184-3523, Agilent Technologies, Palo Alto CA) following the user's manual. Hybridizations were performed using Agilent's Human 1A (V2) Oligo Microarrays (Agilent Technologies, Palo Alto CA) containing about 21,000 unique probes (http://www.agilent.com). The hybridization reference (Cy3) in every case was untransfected cells or a mock transfected cells, as indicated. Slides were washed and dried using 6X and 0.06X SSPE with 0.025% N-lauroylsarcosine and Agilent's proprietary nonaqueous drying and stabilization solution. They were scanned on an Agilent Microarray Scanner (model G2505B) and the raw image was processed using Feature Extraction (v7.5.1).

### C. Statistical Methods

The gene expression data from 2 sets of experiments and multiple microarrays were considered well-measured if the reference channel had > 2 fold of signal intensity over background and was present for > 80% of data set. The data was analyzed using a Rosetta Resolver system (Rosetta Biosoftware, Seattle, USA) . The top genes from each data set were collated, and the expression data of this set of genes from each data set was retrieved and grouped by hierarchical clustering (see, e.g., Eisen et al., Proc. Nat'l Acad. Sci. USA (1998) 95:14863-1486) and displayed in a table format.

### II. RESULTS

A. When comparing transfection experiments, it is important to address the impact of the transfection process itself on the cell. The extent of this impact is dependent on the transfection, can vary between experiments, and can have a drastic impact on readout of the experiment. It thus should be monitored carefully to insure that the outcome of the experiment is due to the intended treatment and not the delivery agent itself.
1. Two samples that are identical but labeled differentially should not show differential regulation. Any differences observed are an indication of genes affected due to random or systematic variations. When we compared two identical mock-transfected samples labeled differentially in Cy3 or Cy5, we observed n= 114 probes that were significantly different i.e up or downregulated at p value cutoff of 0.01. This suggests that labeling itself leads to random differential calls that are due to noise, but this is an extremely small number of cases compared to roughly 22,000 genes interrogated on the array (0.005%).
2. When several mock samples were obtained from independent transfections and were hybridized to one another (sample 2 versus sample 1, sample 3 versus sample 1, and the like), we noticed differences in gene expression reflecting variable effects of the lipid transfection reagent depending on the transfection. Of 4 independent mock transfections studied, mock transfections 1 and 3 were similar to each other while mock transfections 2 and 4 were similar to each other. To determine this, we used mock 1 as the baseline (because mock 1 was common to all comparisons, we calculated ratios (i.e red over green)) and used red as mock 1 and the other channel as the mock-2, -3 or -4. We noticed many genes were differently expressed as a consequence of lipid exposure, where the number of these genes was approximately 200 to 1000. As we measure ratios we are only able to tell the maximal changes (e.g., 1054 genes that were altered between mock 4 and mock 1). It is possible that many other genes were not sampled. However the number 200-1000 is greater than 114, thus these differences are attributed to a non-noise component. By selecting all the genes that are differentially expressed between mock 1 and the other mock-transfected samples but removing the 114 genes that are attributable to a noise component, we identified 1201 genes. Then we clustered using a filtered gene set that consisted of ratios across multiple pairwise comparison. We excluded the mock 1 vs mock 1 pairwise comparison. The hierarchical clustering approaches showed that samples labeled 1 and 3 are related closely to each other and more so than 2 and 4. This suggests there are patterns and commonalities of gene sets that a particular transfection exhibits and is reflective of day-to-day variations in transfection effects that must be monitored and excluded from gene expression analysis of experiments involving delivery of cargo molecules.
3. A large number of the genes were related to inflammatory responses or stress or interferon response pathway in the pairwise comparison. 4. When we compared dye flipped replicates between different mock samples (mock 1 vs 2) labeled in two channels, we observed 198 probes that were consistently differentially expressed. This strongly indicates that these 198 genes for the pairwise comparison are reflective of true biological effects.
5. See the list of genes in Table 1 for genes altered between mock transfections and Table 2 A and 2B for genes upregulated in mock transfections as opposed to the untransfected state.
6. Tables 2A and 2B show that there is differential expression between mock transfected and untransfected cells. Many of these genes overlap between the genes discovered to be differentially affected by independent lipid treatments. This further confirms that treatment by lipid can alter the physiological state of the cell in a moderate to drastic manner. Variation between mock (Table 1) shows that there are transfection specific effects that vary from transfection to transfection and that these expression effects can be used to monitor the degree of cellular perturbation inflicted n the cell by the transfection process.
B. Tables

**TABLE 1. Mock vs. Mock genes differentially regulated at 2-fold.**

| Sequence Code; Sequence Name(s); Accession # | Cy3 Mock1 vs. Cy5 Mock2 | Cy3 Mock4 vs. Cy5 Mock1 | Cy3 Mock2 vs. Cy5 Mock1 | Cy3 Mock3 vs. Cy5 Mock1 |
|---|---|---|---|---|
| NM_152703.1,FLJ39885,I_930405 | 0.67 | 0.82 | 0.61 | 0.26 |
| P80188,LCN2,I_931105 | 0.6 | 0.75 | 0.53 | 0.25 |
| 013113,DD96,I_1221815 | 0.6 | 0.72 | 0.43 | 0.29 |
| NM_006074.2,TRIM22,I_930081 | 0.76 | 0.85 | 0.61 | 0.41 |
| AAC50161.1,G1P3,I_928835 | 0.59 | 0.76 | 0.66 | 0.4 |
| P05305.EDN1,I_957098 | 0.61 | 0.78 | 0.64 | 0.46 |
| P20592,MX2,I_962329 | 0.55 | 0.75 | 0.59 | 0.37 |
| NM_000331.1,NM_000331.1.NM_000331.1 | 0.62 | 0.7 | 0.69 | 0.36 |
| P05231,IL6,I_929482 | 0.55 | 0.63 | 0.6 | 0.4 |
| 070303,I_1109441,I_1109441 | 0.15 | 0.33 | 0.31 | 0.2 |
| P05937,CALB1,I_929807 | 0.13 | 0.31 | 0.32 | 0.22 |
| Q9Y6Q1,CAPN6,I_966236 | 0.13 | 0.35 | 0.31 | 0.25 |
| AAC78554.1,SULT2B1,I_962443 | 0.19 | 0.31 | 0.33 | 0.25 |
| CAD29001.1,ERK8,I_964782 | 0.19 | 0.3 | 0.31 | 0.23 |
| NM_016562.2,TLR7,I_962503 | 0.08 | 0.39 | 0.33 | 0.24 |
| BAA28263.1,I_962396,I_962396 | 0.08 | 0.38 | 0.36 | 0.26 |
| NM_005116.3.SLC23A1,I_961596 | 0.13 | 0.36 | 0.38 | 0.25 |
| AAH40535.1,KIAA1145,I_943975 | 0.14 | 0.34 | 0.38 | 0.3 |
| NM_152504.1,NM_152504.1,NM_152504.1 | 0.16 | 0.39 | 0.32 | 0.29 |
| Q13361,MAGP2,I_945833 | 0.18 | 0.37 | 0.37 | 0.27 |
| NM_138434.1,NM_138434.1,NM_138434.1 | 0.17 | 0.41 | 0.27 | 0.16 |
| AAL76329.1,OPTN,I_931662 | 0.17 | 0.39 | 0.27 | 0.18 |
| P49716,CEBPD,I_929950 | 0.14 | 0.37 | 0.29 | 0.17 |
| NM_017632.1,CARF,I_963939 | 0.13 | 0.36 | 0.24 | 0.21 |
| AL834442.1,I_957663,I_957663 | 0.16 | 0.32 | 0.23 | 0.15 |
| P48060,GUPR1,I_964747 | 0.18 | 0.31 | 0.25 | 0.18 |
| NM_004244.2,I_964221,I_964221 | 0.12 | 0.3 | 0.26 | 0.18 |
| CAA75245.1,B3GALT2,I_930112 | 0.14 | 0.31 | 0.26 | 0.22 |
| P15260,IFNGR1,I_966769 | 0.23 | 0.35 | 0.28 | 0.22 |
| NM_145176.1,SLC2A12.NM_145176.1 | 0.24 | 0.34 | 0.27 | 0.21 |
| AAH47411.1,NYD-TSPG,I_957046 | 0.23 | 0.33 | 0.3 | 0.23 |
| AAH37574.1,FLJ12552,I_957656 | 0.26 | 0.34 | 0.3 | 0.23 |
| Q9UBP4,DKK3,I_934794 | 0.25 | 0.3 | 0.28 | 0.22 |
| NM_003955.2,SOCS3,NM_003955.2 | 0.22 | 0.31 | 0.3 | 0.21 |
| AAH14081.1,TAP1,I_966923 | 0.2 | 0.31 | 0.29 | 0.14 |
| Q13287,NMI,I_938821 | 0.21 | 0.33 | 0.29 | 0.17 |
| P22680,CYP7A1,I_929723 | 0.21 | 0.34 | 0.22 | 0.18 |
| CAC60189.1,SULF1,I_963018 | 0.25 | 0.38 | 0.21 | 0.18 |
| BAA34533.1,LAMP3,I_928419 | 0.25 | 0.34 | 0.36 | 0.24 |
| Q96DX8,IFRG28,I_944035 | 0.27 | 0.33 | 0.38 | 0.19 |
| P21673,SAT,I_966249 | 0.2 | 0.48 | 0.28 | 0.22 |
| O75556,SCGB2A1,I_931332 | 0.25 | 0.42 | 0.36 | 0.26 |
| P02511,CRYAB,I_932097 | 0.26 | 0.44 | 0.32 | 0.22 |
| P05408,I_963813,I_963813 | 0.22 | 0.47 | 0.38 | 0.21 |
| CAD79908,I_931444,I_931444 | 0.18 | 0.41 | 0.39 | 0.22 |
| P21580,TNFAIP3,I_966771 | 0.28 | 0.48 | 0.3 | 0.29 |
| NM_024827.1,HDAC11,I_928610 | 0.18 | 0.29 | 0.29 | 0.37 |
| Q9UHA7,I_942165,I_942165 | 0.05 | 0.31 | 0.23 | 0.2 |
| O75912,DGKI,I_1100395 | 0.09 | 0.34 | 0.24 | 0.2 |
| NM_145699.2,APOBEC3A,NM_145699.2 | 0.04 | 0.35 | 0.27 | 0.17 |
| AAD31386.1,LHFP,I_960816 | 0.09 | 0.33 | 0.27 | 0.25 |
| P25090,FPRL1,I_966360 | 0.05 | 0.33 | 0.25 | 0.31 |
| NM_015242.1,I_931000,I_931000 | 0.07 | 0.32 | 0.36 | 0.2 |
| Q92908,I_960645,_960645 | 0.07 | 0.33 | 0.35 | 0.19 |
| CAC25091.1,I_1152083,I_1152083 | 0.06 | 0.33 | 0.34 | 0.25 |
| P22223,CDH3,I_959174 | 0.03 | 0.3 | 0.3 | 0.2 |
| NM_004209.4,SYNGR3,I_959220 | 0.08 | 0.26 | 0.34 | 0.14 |
| O60268,KIAA0513,I_959869 | -0.08 | 0.28 | 0.31 | 0.19 |
| NM_058186.2,FAM3B,I_962328 | -0.05 | 0.31 | 0.26 | 0.2 |
| P15822,HIVEP1,I_957097 | -0.03 | 0.31 | 0.28 | 0.16 |
| I_929263,I_929263,I_929263 | 0.05 | 0.39 | 0.35 | 0.34 |
| NM_024073.1,MGC2875,I_962694 | 0.03 | 0.34 | 0.39 | 0.31 |
| NM_024709.1,FLJ14146,I_929327 | 0.09 | 0.35 | 0.4 | 0.34 |
| Q16517,NNAT,I_961363 | -0.02 | 0.33 | 0.32 | 0.3 |
| AAH14949.1,LGP2,I_960045 | 0.18 | 0.32 | 0.2 | 0.09 |
| NM_003358.1,NM_003358.1,NM_003358.1 | 0.19 | 0.32 | 0.19 | 0.07 |
| NM_004900.3,APOBEC3B,I_961947 | 0.17 | 0.33 | 0.26 | 0.09 |
| P01884,B2M,I_958944 | 0.2 | 0.32 | 0.24 | 0.13 |
| P16870,CPE,I_957382 | 0.09 | 0.32 | 0.2 | 0.09 |
| P24821,TNC,I_931261 | 0.23 | 0.31 | 0.14 | 0.1 |
| NM_005711.2,EDIL3,NM_005711.2 | 0.18 | 0.31 | 0.1 | 0.15 |
| P09913,IFIT2,I_931455 | 0.18 | 0.44 | 0.2 | 0.02 |
| Q99715,COL12A1,I_957267 | 0.05 | 0.42 | 0.24 | 0.08 |
| Q9Y3Z3,SAMHD1,I_961367 | 0.3 | 0.42 | 0.4 | 0.17 |
| P32455,GBP1,I_929551 | 0.32 | 0.4 | 0.38 | 0.18 |
| NM_003335.1,UBE1L,I_965485 | 0.31 | 0.4 | 0.35 | 0.17 |
| P19876,CXCL3,I_957616 | 0.35 | 0.41 | 0.39 | 0.17 |
| AAH12125.1,BIGM103,I_966093 | 0.27 | 0.38 | 0.33 | 0.18 |
| P09486,SPARC,I_957731 | 0.31 | 0.4 | 0.35 | 0.12 |
| P00736,C1R,I_963185 | 0.32 | 0.47 | 0.33 | 0.18 |
| Q13145,NMA,I_931547 | 0.31 | 0.35 | 0.29 | 0.17 |
| NM_018370.1,FLJ11259,I_933020 | 0.32 | 0.35 | 0.27 | 0.21 |
| O95832,CLDN1,I_928765 | 0.36 | 0.38 | 0.33 | 0.17 |
| NM_032866.1.FLJ14957,I_1000503 | 0.33 | 0.37 | 0.34 | 0.18 |
| NM_006820.1.C1orf29,I_939428 | 0.35 | 0.35 | 0.31 | 0.17 |
| Q13325,RI58,I_931458 | 0.35 | 0.34 | 0.28 | 0.12 |
| P05161,G1P2,I_938569 | 0.31 | 0.39 | 0.29 | 0.13 |
| NM_018284.1,GBP3,I_1221809 | 0.34 | 0.38 | 0.28 | 0.14 |
| Q13489, BIRC3,I_930289 | 0.38 | 0.4 | 0.29 | 0.21 |
| AAF34183.1,I_1100498,I_1100498 | 0.33 | 0.42 | 0.33 | 0.22 |
| Q9C002,NMES1,I_958605 | 0.35 | 0.4 | 0.36 | 0.26 |
| NM_002185.2,IL7R,NM_002185.2 | 0.43 | 0.43 | 0.31 | 0.16 |
| NM_145640.1,APOL3,I_961472 | 0.28 | 0.43 | 0.26 | 0.1 |
| NM_005132.1,NM_005132.1,NM_005132.1 | 0.24 | 0.47 | 0.25 | 0.13 |
| Q00978,ISGF3G,I_958873 | 0.23 | 0.38 | 0.27 | 0.1 |
| BAA88519.1,CEB1,I_957526 | 0.22 | 0.39 | 0.26 | 0.11 |
| NM_144975.1,NM_144975.1,NM_144975.1 | 0.26 | 0.51 | 0.25 | 0.17 |
| AL137572.1,I_1985061.FL1,I_1985061.FL1 | 0.3 | 0.52 | 0.26 | 0.13 |
| P09871,C1S,I_936464 | 0.29 | 0.44 | 0.26 | 0.18 |
| Q06828,FMOD,I_929994 | 0.35 | 0.47 | 0.24 | 0.1 |
| BAC23101.1,FLJ20073,I_930406 | 0.46 | 0.47 | 0.25 | 0.09 |
| NM_001549.1,IFIT4,I_931456 | 0.36 | 0.43 | 0.24 | 0.02 |
| P19525,PRKR,I_936379 | 0.4 | 0.41 | 0.26 | 0.06 |
| NM_004848.1.C1orf38,I_1002342 | 0.36 | 0.33 | 0.36 | 0.07 |
| Q99814,EPAS1,I_962988 | 0.25 | 0.25 | 0.31 | 0.18 |
| P04270,ACTC,I_959559 | 0.27 | 0.22 | 0.31 | 0.16 |
| AAH11454.1,I_943630,I_943630 | 0.31 | 0.28 | 0.32 | 0.15 |
| Q16739,UGCG,I_930754 | 0.32 | 0.36 | 0.13 | 0.18 |
| P36959,GMPR,I_966598 | 0.35 | 0.2 | 0.18 | 0.08 |
| P13501,CCL5,I_960100 | 0.34 | 0.25 | 0.18 | 0.07 |
| Q12929,EPS8,I_966027 | 0.3 | 0.3 | 0.19 | 0.14 |
| Q96CA5,BIRC7,I_966160 | 0.31 | 0.24 | 0.22 | 0.18 |
| NM_005531.1,I_935121,I_935121 | 0.36 | 0.32 | 0.21 | 0.06 |
| AAO15881.1,1 957576,1 957576 | 0.36 | 0.32 | 0.22 | 0.13 |
| NM_005101.1.NM_005101.1.NM_005101.1 | 0.37 | 0.34 | 0.22 | 0.1 |
| CAD57238.1,PCTAIRE2BP,1_1152159 | 0.29 | 0.32 | 0.21 | 0.05 |
| P03996.ACTA2,I_931577 | 0.39 | 0.28 | 0.36 | 0.19 |
| O00622,CYR61,I_931923 | 0.5 | 0.28 | 0.37 | 0.21 |
| Q99988,PLAB,I_966585 | 0.44 | 0.22 | 0.35 | 0.1 |
| Q9BZQ8, C1or 24,I_928963 | 0.38 | 0.3 | 0.09 | 8.05E-05 |
| NM_003733.1,OASL,I_935660 | 0.32 | 0.31 | 0.2 | -0.03 |
| NM_017912.1,FLJ20637,I_957527 | 0.36 | 0.56 | 0.39 | 0.22 |
| P09914,IFIT1,I_931457 | 0.41 | 0.53 | 0.37 | 0.22 |
| NM_031458.1,BAL,I_943932 | 0.35 | 0.51 | 0.4 | 0.25 |
| NM_030754.2,SAA2,NM_030754.2 | 0.4 | 0.48 | 0.39 | 0.27 |
| P27469,GOS2,I_939253 | 0.37 | 0.62 | 0.42 | 0.2 |
| AAD19826.1,RIG-I,I_930876 | 0.37 | 0.58 | 0.42 | 0.16 |
| NM_025079.1.NM_025079.1,NM_025079.1 | 0.3 | 0.51 | 0.45 | 0.3 |
| P01584,IL 1B,I_942167 | 0.34 | 0.52 | 0.45 | 0.3 |
| NM_001733.1,I_964222,I_964222 | 0.24 | 0.59 | 0.42 | 0.22 |
| P00751.BF,I_1109725 | 0.49 | 0.57 | 0.3 | 0.09 |
| BAA02837.1,OSF-2,I_963643 | 0.45 | 0.67 | 0.36 | 0.21 |
| AB095925.1,I_2026991.FL1,I_2026991.FL1 | 0.45 | 0.49 | 0.4 | 0.14 |
| P13500,CCL2,I_959180 | 0.46 | 0.45 | 0.43 | 0.07 |
| P19875,CXCL2,I_957614 | 0.43 | 0.46 | 0.47 | 0.2 |
| P09341 ,CXCL1,I_957623 | 0.48 | 0.39 | 0.43 | 0.14 |
| P42224.STAT1,I_938024 | 0.49 | 0.43 | 0.42 | 0.17 |
| P10145,IL8,I_957620 | 0.49 | 0.42 | 0.4 | 0.23 |
| Q9UMW8,USP18,I_960965 | 0.56 | 0.53 | 0.42 | 0.12 |
| P00973,OAS1,I_934667 | 0.57 | 0.41 | 0.44 | 0.11 |
| P13164,IFITM1,I_965186 | 0.45 | 0.62 | 0.56 | 0.29 |
| NM_002089.1,NM_002089.1.NM_002089.1 | 0.47 | 0.63 | 0.58 | 0.31 |
| NM 006187.1,OAS3,1_1109804 | 0.49 | 0.61 | 0.55 | 0.27 |
| P04179,SOD2,I_956965 | 0.44 | 0.53 | 0.45 | 0.31 |
| P51911,CNN1,I_1221748 | 0.49 | 0.54 | 0.41 | 0.26 |
| O15162,PLSCR1,I_928693 | 0.53 | 0.53 | 0.49 | 0.28 |
| P12718,ACTG2,I_947174 | 0.64 | 0.59 | 0.46 | 0.24 |
| CAA68168.1,CCL8,I_959183 | 0.55 | 0.54 | 0.55 | 0.15 |
| NM_016582.1,NM_016582.1,NM_016582.1 | 0.6 | 0.57 | 0.6 | 0.22 |
| AAG34368.1,MDA5,I_941111 | 0.4 | 0.67 | 0.75 | 0.16 |
| NM_024557.2,FLJ11608,I_930538 | -0.11 | 0.53 | 0.32 | 0.39 |
| AAD04726.1,I_929599,I_929599 | -0.06 | 0.48 | 0.4 | 0.34 |
| NM_014926.1,KIAA0848,I_956933 | 0.02 | 0.46 | 0.36 | 0.33 |
| I_962738,I_962738,I_962738 | -0.07 | 0.41 | 0.37 | 0.36 |
| P45844,ABCG 1, I_962066 | -0.1 | 0.35 | 0.38 | 0.39 |
| NM_024070.1,MGC2463,I_929580 | 0.06 | 0.46 | 0.44 | 0.4 |
| NM_052913.1 ,NM_052913.1 ,NM_052913.1 | 0 | 0.4 | 0.5 | 0.43 |
| O95990,TU3A,I_963407 | -0.19 | 0.54 | 0.46 | 0.37 |
| NM_145024.1,FLJ31547,I_959621 | 0.13 | 0.63 | 0.51 | 0.45 |
| NM_031218.1,FLJ12488,I_965064 | -0.07 | 0.63 | 0.57 | 0.55 |
| P05814,CSN2,I_957737 | -0.03 | 0.56 | 0.54 | 0.45 |
| AAH21089.1,PPP1R14A,I_966136 | 0.18 | 0.47 | 0.57 | 0.4 |
| P26022,PTX3,I_928369 | 0.2 | 0.5 | 0.49 | 0.39 |
| Q16676,FOXD1,I_957586 | -0.23 | 0 | -0.07 | 0.35 |
| NM_001956.1,NM_001956.1,NM_001956.1 | -0.32 | | -0.24 | 0.41 |
| AK001520.1,I_958247,I_958247 | -0.07 | 0.4 | 0.1 | 0.35 |
| BAA24854.1,ARHGEF9,I_962149 | -0.04 | 0.23 | 0.11 | 0.31 |
| NM_005060.2.RORC.NM_005060.2 | 0.01 | 0.21 | 0.23 | 0.3 |
| Q9NR23,GDF3,I_1109802 | 0.01 | 0.21 | 0.22 | 0.31 |
| I_959946,I_959946,I_959946 | -0.01 | 0.26 | -0.14 | 0.4 |
| AAH22324.1,MGC22679,I_1100079 | -0.02 | 0.07 | 0.12 | 0.81 |
| Q9Y5L2,HIG2,I_930773 | -0.33 | -0.24 | -0.28 | 0.1 |
| P49759,CLK1,I_932067 | -0.35 | -0.1 | -0.28 | -0.01 |
| NM_152392.1,AHSA2,NM_152392.1 | -0.29 | -0.13 | -0.3 | -0.01 |
| AAH15236.1,RTP801,I_932259 | -0.25 | -0.32 | -0.37 | -0.18 |
| NM_004750.2,CRLF1,I_966345 | -0.29 | -0.39 | -0.34 | -0.21 |
| Q16790,CA9,I_1110179 | -0.35 | -0.32 | -0.28 | -0.15 |
| AAK67646.1,ADSSL1,I_959378 | -0.37 | -0.33 | -0.37 | -0.21 |
| NM_003546.2,HIST1H4L,I_958017 | -0.12 | -0.31 | -0.16 | -0.31 |
| NM_003495.2,NM_003495.2,NM_003495.2 | -0.14 | -0.32 | -0.17 | -0.32 |
| NM_003544.2,HIST1H4B,I_957901 | -0.06 | -0.32 | -0.16 | -0.26 |
| NM_022908.1,FLJ12442,I_965411 | -0.18 | -0.37 | -0.27 | -0.27 |
| CAD39167.1,MacGAP,I_957035 | -0.09 | -0.36 | -0.25 | -0.19 |
| AAH05847.1,I_963110,I_963110 | -0.1 | -0.16 | -0.18 | -0.34 |
| NM_004324.2,BAX,I_962422 | -0.02 | -0.13 | -0.21 | -0.33 |
| NM_004890.1,SPAG7,I_960716 | 0.03 | -0.32 | -0.26 | -0.19 |
| NM_145080.1,NSE1,I_963635 | 0.02 | -0.31 | -0.2 | -0.15 |
| Q01844,EWSR1,I_961280 | 0.07 | -0.34 | -0.21 | -0.2 |
| AAH00655.1,DKFZP586F1524,I_960228 | 0.06 | -0.33 | -0.16 | -0.19 |
| P08107,HSPA1A,I_1152464 | 0.12 | -0.38 | -0.07 | -0.24 |
| CAA28352.1,SNRP70,I_961545 | 0.15 | -0.36 | -0.17 | -0.26 |
| P45974,USP5,I_936453 | 0.12 | -0.31 | -0.12 | -0.22 |
| P49411,I_1201761,I_1201761 | 0.1 | -0.39 | -0.22 | -0.32 |
| P14648,SNRPN,I_958536 | 0.11 | -0.45 | -0.25 | -0.29 |
| Q9ULX6,NAKAP95,I_961808 | 0.17 | -0.27 | -0.28 | -0.32 |
| NM_003765.1,NM_003765.1,NM_003765.1 | 0.12 | -0.3 | -0.26 | -0.25 |
| NM_012404.2,ANP32D,I_965845 | 0.16 | -0.3 | -0.26 | -0.07 |
| P20591,MX1,I_962330 | 0.89 | 1.16 | 0.94 | 0.34 |
| P40305,IFI27,I_959599 | 1.02 | 1.29 | 1.07 | 0.42 |

**TABLE 2 A. Genes Upregulated in Mock transfection lipid**

| Sequence Code | Sequence Name(s) | Accession # | Log(Ratio) | Ratio | P-value |
|---|---|---|---|---|---|
| P09341 | GRO1 | I_957623 | -0.84 | 0.14 | 2.14E-07 |
| P19876 | GRO3 | I_957616 | -0.57 | 0.27 | 4.86E-22 |
| P05161 | ISG15 | I_938569 | -0.56 | 0.27 | 0 |
| AAH22367.1 | FLJ14440 | I_957666 | -0.56 | 0.28 | 8.42E-34 |
| AAH04179.1 | MGC2780 | I_963730 | -0.54 | 0.29 | 2.49E-26 |
| P09913 | IFIT2 | I_931455 | -0.52 | 0.3 | 2.57E-22 |
| AAB53416.1 | ISG20 | I_962689 | -0.48 | 0.33 | 1.63E-38 |
| P09601 | HMOX1 | I_961390 | -0.48 | 0.33 | 3.49E-13 |
| AAD19826.1 | RIG-I | I_930876 | -0.47 | 0.34 | 2.27E-23 |
| O14879 | IFIT4 | I_931456 | -0.44 | 0.37 | 8.60E-22 |
| Q15646 | OASL | I_935660 | -0.43 | 0.37 | 0 |
| Q16719 | KYNU | I_1002115 | -0.42 | 0.38 | 2.04E-13 |
| Q99988 | PLAB | I_966585 | -0.42 | 0.38 | 7.75E-31 |
| P19875 | GRO2 | I_957614 | -0.4 | 0.4 | 7.54E-03 |
| P48745 | NOV | I_929821 | -0.4 | 0.4 | 1.91E-16 |
| AAH30039.1 | I_1000634 | I_1000634 | -0.38 | 0.42 | 6.80E-12 |
| P29121 | I_1110326 | I_1110326 | -0.38 | 0.41 | 5.46E-24 |
| CAD35098.1 | IF144 | I_939429 | -0.37 | 0.43 | 8.25E-23 |
| P24385 | CCND1 | I_929264 | -0.37 | 0.43 | 1.73E-15 |
| BAA88519.1 | LOC51191 | I_957526 | -0.36 | 0.44 | 5.73E-29 |
| 076061 | STC2 | I_957249 | -0.36 | 0.44 | 1.58E-12 |
| P05231 | IL6 | I_929482 | -0.35 | 0.45 | 3.35E-07 |
| P15407 | FOSL1 | I_1110036 | -0.34 | 0.45 | 4.95E-11 |
| P48506 | GCLC | I_957559 | -0.34 | 0.46 | 6.52E-16 |
| Q01201 | RELB | I_960902 | -0.34 | 0.46 | 2.45E-17 |
| P18847 | ATF3 | I_929428 | -0.33 | 0.47 | 6.44E-09 |
| 095084 | SPUVE | I_1221819 | -0.32 | 0.47 | 1.84E-08 |
| AAH16836.1 | SQRDL | I_963362 | -0.32 | 0.48 | 6.00E-15 |
| P52895 | AKR1C2 | I_963167 | -0.32 | 0.48 | 9.28E-14 |
| Q99467 | LY64 | I_957360 | -0.31 | 0.49 | 9.59E-04 |
| AAH27612.1 | I_1100631 | I_1100631 | -0.31 | 0.49 | 1.44E-21 |
| CAB81634.1 | C20orf97 | I_961961 | -0.31 | 0.49 | 1.13E-06 |
| AAF61195.1 | FLB6421 | I_929296 | -0.3 | 0.5 | 1.98E-12 |
| P20591 | MX1 | I_962330 | -0.29 | 0.51 | 2.88E-16 |
| AAC06022.1 | I_1151917 | I_1151917 | -0.29 | 0.52 | 0.02 |
| Q99814 | EPAS1 | I_962988 | -0.28 | 0.53 | 2.69E-15 |
| P08174 | DAF | I_931297 | -0.28 | 0.53 | 7.52E-04 |
| AAH14103.1 | I_946379 | I_946379 | -0.27 | 0.54 | 2.85E-13 |
| AAC52070.1 | SQSTM1 | I_957549 | -0.27 | 0.54 | 7.95E-08 |
| P49767 | VEGFC | I_957216 | -0.27 | 0.53 | 1.69E-04 |
| AAL77033.1 | I_1100868 | I_1100868 | -0.27 | 0.54 | 1.19E-25 |
| Q9Y5E4 | PCDHB5 | I_956984 | -0.27 | 0.54 | 6.96E-03 |
| AAH04107.1 | FST | I_957313 | -0.26 | 0.55 | 2.72E-10 |
| P11021 | HSPA5 | I_930977 | -0.26 | 0.55 | 7.15E-05 |
| P48507 | GCLM | I_936692 | -0.26 | 0.55 | 1.02E-12 |
| P02792 | FTL | I_1002418 | -0.26 | 0.55 | 1.49E-23 |
| AAK07684.1 | TBC1D2 | I_1152495 | -0.25 | 0.56 | 5.00E-05 |
| AAC72344.1 | IER3 | I_1110256 | -0.25 | 0.56 | 1.28E-07 |
| P42330 | AKR1C3 | I_932290 | -0.25 | 0.56 | 1.46E-12 |
| P07093 | SERPINE2 | I_1000015 | -0.24 | 0.58 | 0.02 |
| BAA91303.1 | FLJ20637 | I_957527 | -0.23 | 0.59 | 5.24E-06 |
| AAD28245.1 | BCAR3 | I_936333 | -0.23 | 0.59 | 1.08E-43 |
| BAA21824.1 | LRP8 | I_929104 | -0.23 | 0.6 | 1.42E-04 |
| BAA91809.1 | I_1002443 | I_1002443 | -0.23 | 0.59 | 3.16E-22 |
| P51843 | NROB1 | I_962357 | -0.23 | 0.59 | 1.41E-23 |
| 095415 | BRI3 | I_928931 | -0.23 | 0.59 | 2.02E-11 |
| Q969L2 | MAL2 | I_929990 | -0.22 | 0.6 | 1.09E-08 |
| Q13489 | BIRC3 | I_930289 | -0.22 | 0.6 | 9.40E-08 |
| BAA31969.1 | DUSP6 | I_932638 | -0.21 | 0.62 | 6.95E-04 |
| 094907 | DKK1 | I_932501 | -0.21 | 0.62 | 4.17E-08 |
| Q14956 | GPNMB | I_929480 | -0.21 | 0.62 | 3.75E-13 |
| BAA91772.1 | FLJ10724 | I_965603 | -0.21 | 0.61 | 5.24E-07 |
| P30408 | TM4SF1 | I_965968 | -0.21 | 0.62 | 3.21E-06 |
| Q13794 | PMAIP1 | I_964520 | -0.21 | 0.62 | 2.40E-07 |
| P30405 | PPIF | I_931912 | -0.2 | 0.64 | 5.75E-07 |
| P29275 | ADORA2B | I_958524 | -0.2 | 0.63 | 8.62E-07 |
| P23560 | BDNF | I_934604 | -0.2 | 0.63 | 8.27E-05 |
| BAA91172.1 | FLJ20442 | I_965681 | -0.2 | 0.64 | 7.40E-05 |
| AB037784.1 | I_944462 | I_944462 | -0.2 | 0.64 | 2.58E-19 |

**TABLE 2B. Genes Downregulated in Mock transfection lipid**

| Sequence Code | Sequence Name(s) | Accession # | Log(Ratio) | Ratio | P-value |
|---|---|---|---|---|---|
| P05787 | KRT8 | I_1002120 | 0.2 | 1.58 | 4.03E-05 |
| BAA91265.1 | FLJ20568 | I_959519 | 0.2 | 1.59 | 6.70E-04 |
| P10827 | THRA | I_960982 | 0.2 | 1.57 | 7.64E-11 |
| P98082 | DAB2 | I_958462 | 0.2 | 1.58 | 5.25E-03 |
| AAH12625.1 | PPP1R3C | I_932078 | 0.2 | 1.6 | 1.17E-11 |
| Q03591 | HFL1 | I_928792 | 0.2 | 1.6 | 7.73E-06 |
| P50238 | CRIP1 | I_964001 | 0.2 | 1.59 | 1.98E-04 |
| I_931924 | I_931924 | I_931924 | 0.21 | 1.63 | 0.55 |
| Q12796 | PROL2 | I_964640 | 0.21 | 1.62 | 2.22E-17 |
| P55268 | LAMB2 | I_1000561 | 0.21 | 1.64 | 1.09E-09 |
| AAF78243.1 | SEC31B-1 | I_931537 | 0.21 | 1.63 | 1.45E-09 |
| Q99489 | DDO | I_957497 | 0.21 | 1.61 | 1.65E-05 |
| AAH11405.1 | I_1100083 | I_1100083 | 0.22 | 1.64 | 9.37E-04 |
| Q16612 | I_957423 | I_957423 | 0.22 | 1.66 | 1.17E-04 |
| AAF04336.1 | RGC32 | I_1109905 | 0.22 | 1.68 | 5.84E-10 |
| P35241 | RDX | I_931169 | 0.22 | 1.67 | 2.86E-09 |
| P47895 | ALDH1A3 | I_959908 | 0.22 | 1.67 | 7.76E-03 |
| P10589 | NR2F1 | I_957262 | 0.23 | 1.71 | 1.14E-08 |
| P53420 | COL4A4 | I_1221788 | 0.23 | 1.72 | 2.06E-07 |
| P51884 | LUM | I_932496 | 0.24 | 1.73 | 1.75E-08 |
| P02461 | COL3A1 | I_928318 | 0.24 | 1.76 | 1.14E-08 |
| BC019351.1 | I_957786 | I_957786 | 0.25 | 1.76 | 0.03 |
| Q9UKW4 | VAV3 | I_963191 | 0.25 | 1.77 | 8.00E-09 |
| BAA86561.2 | KIAA1247 | I_962934 | 0.25 | 1.79 | 2.92E-10 |
| BC015670.1 | I_958949 | I_958949 | 0.26 | 1.82 | 1.74E-08 |
| P20742 | PZP | I_932541 | 0.26 | 1.83 | 2.14E-05 |
| P78334 | GABRE | I_959453 | 0.27 | 1.85 | 1.05E-15 |
| AAH26104.1 | PDCD4 | I_1221853 | 0.27 | 1.86 | 1.36E-08 |
| 060437 | PPL | I_959240 | 0.27 | 1.87 | 2.20E-07 |
| CAD34911.1 | I_928640 | I_928640 | 0.27 | 1.86 | 2.90E-06 |
| Q16621 | NFE2 | I_932056 | 0.28 | 1.89 | 1.03E-18 |
| P01023 | A2M | I_932542 | 0.28 | 1.92 | 6.48E-05 |
| P50748 | KNTC1 | I_931698 | 0.28 | 1.89 | 2.32E-10 |
| P55001 | MFAP2 | I_931388 | 0.29 | 1.97 | 1.72E-23 |
| 000158 | LMO4 | I_928768 | 0.29 | 1.96 | 1.12E-26 |
| CAA25086.1 | FTH1 | I_931392 | 0.29 | 1.93 | 9.94E-05 |
| P25800 | LMO1 | I_930540 | 0.29 | 1.94 | 8.16E-16 |
| BAB15690.1 | FLJ23550 | I_933953 | 0.3 | 1.99 | 1.89E-03 |
| AAM33633.1 | I_928737 | I_928737 | 0.32 | 2.09 | 3.03E-05 |
| AAA60095.1 | PRKCB1 | I_959615 | 0.32 | 2.1 | 1.96E-36 |
| Q14050 | COL9A3 | I_961783 | 0.34 | 2.21 | 2.99E-12 |
| AAG42072.1 | IDAX | I_957995 | 0.34 | 2.2 | 2.65E-10 |
| P08727 | KRT19 | I_1110001 | 0.43 | 2.68 | 9.98E-12 |
| AAA97890.1 | PDE4D | I_1221771 | 0.47 | 2.92 | 1.69E-04 |

All publications, patents, and patent applications cited in this specification are herein incorporated by reference as if each individual publication or patent application were specifically and individually indicated to be incorporated by reference. The citation of any publication is for its disclosure prior to the filing date and should not be construed as an admission that the present invention is not entitled to antedate such publication by virtue of prior invention. Nothing herein is to be construed as an admission that the present invention is not entitled to antedate such publication by virtue of prior invention. Further, the dates of publication provided may be different from the actual publication dates which may need to be independently confirmed.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

The disclosures in United States patent application nos. 11/059,209 and 11/150,409 from which this application claims priority, and in the abstract accompanying this application are incorporated herein by reference.

## Claims

1. A method of determining whether a transfection agent-mediated cytotoxic response has occurred in a cell, said method including:
(a) contacting said cell with a transfection agent; and
(b) evaluating a cytotoxic marker profile of said cell to determine whether said transfection agent-mediated cytotoxic response has occurred in said cell.

2. A method as claimed in claim 1, wherein said cytotoxic marker profile includes data for at least two cytotoxic markers.

3. A method as claimed in claim 1 or 2, wherein said at least one cytotoxic marker is expressed by a gene listed in Table 1.

4. A method as claimed in claim 3, wherein said expression profile is a nucleic acid expression profile.

5. A method as claimed in claim 3, wherein said expression profile is a polypeptide expression profile.

6. A method as claimed in any preceding claim, wherein said transfection agent is a lipid-based transfection agent.

7. A method as claimed in any preceding claim, wherein said method is employed to evaluate data obtained from a gene-silencing assay that includes contacting a cell with a gene-silencing agent and observing said cell for a phenotypic change.

8. An array of probes immobilised on a solid support, said array including:
at least one transfection agent mediated non-specific cytotoxicity probe that specifically binds to an expression product of a gene listed in Table 1.

9. A kit including:
a probe for evaluating a cytotoxic marker associated with transfection agent mediated non-specific cytotoxicity.

10. A collection of gene-specific primers including primers specific for at least two genes listed in Table 1.
